# EUROPEAN PATENT SPECIFICATION

(11) **EP 4 117 744 B1**
(45) Date of publication and mention of the grant of the patent: **17.12.2025**
(21) Application number: 21710511.3
(22) Date of filing: 12.03.2021
(51) Int. Cl.: A61M 1/00

(54) **APPARATUS FOR NEGATIVE PRESSURE TREATMENT AND INSTILLATION OF WOUNDS**
VORRICHTUNG ZUR UNTERDRUCKBEHANDLUNG UND INSTILLATION VON WUNDEN
DISPOSITIF DE TRAITEMENT SOUS PRESSION ET D'INSTILLATION DE PLAIES

(30) Priority: 13.03.2020 EP 20163026
(43) Date of publication of application: 18.01.2023
(73) Proprietor: MEDAXIS AG, 6340 Baar (CH)
(72) Inventor: BANNWART, Lukas, 6343 Rotkreuz (CH)
(74) Representative: Grünecker Patent- und Rechtsanwälte PartG mbB
(86) International application number: PCT/EP2021/056341
(87) International publication number: WO 2021/180926

(56) References cited:
- EP-A2- 0 880 953
- DE-U1- 202017 003 262
- US-B2- 7 338 482

## Description

### TECHNICAL FIELD

The present invention relates to an apparatus for negative pressure treatment and instillation of wounds. The invention also relates to a method for operating such an apparatus.

### PRIOR ART

It has long been known in the medical field that wound healing can be improved by using negative pressure treatment of the wound bed, in particular in the case of large and/or difficult to heal wounds (so-called NPWT - negative-pressure wound therapy). Body fluids or secretions, which are also known as exudate, are extracted from the wound bed by way of a pump. During the negative pressure treatment, wound healing can also be positively influenced by supplying instillation fluid to the wound bed. Such methods are known as negative pressure wound therapy with combined instillation or irrigation. Depending on the type and size of the wound, the extraction and supply take place simultaneously, successively and/or alternately intermittently.

The instillation fluid to be supplied can be, for example, a physiological or non-physiological saline solution, a medicinal product, or a mixture thereof. The instillation fluid can be used, for example, to promote wound healing, to prevent infections, for local anesthesia, or to improve blood coagulation by inhibiting fibrinolysis. The supply of the instillation fluid can therefore be used for rinsing or therapeutic, diagnostic and/or preventive purposes.

General descriptions of the combined negative pressure and instillation treatment of wounds can be found, for example, in the publication Bobkiewicz, Adam et al.; "Negative pressure wound therapy with instillation (NPWTi): Current Status, recommendations and perspectives in the context of modern wound therapy"; "Negative Pressure Wound Therapy Joumal", [S.I.], v. 3, p. 1, Apr. 2016; or in the publication Gupta, Subhas et al.; "Clinical recommendations and practical guide for negative pressure wound therapy with instillation"; "Int Wound J", 2016 Apr; 13(2): 159-174.

For supplying the instillation fluid, a liquid bag or bottle filled with the fluid to be supplied is often arranged elevated above the body part to be treated, similar to conventional infusion, so that the fluid is supplied to the part to be treated through a supply line due to the hydrostatic pressure. Apart from this, the body fluids are extracted by a vacuum pump via a respective line.

In order to enable better adjustment and control when supplying the instillation fluid, and/or to be independent of the arrangement and in particular the height level of the fluid container filled with the fluid, systems are also well known in which the supply of the fluid to the body is effected by a pump, in particular a so-called peristaltic or tube pump.

To monitor the therapy, the pressure prevailing in the wound area is measured continuously or at regular time intervals in many prior art systems. This is achieved, for example, by use of an auxiliary line which opens laterally into the secretion line in the region of its end at the patient side, or which is passed through the wound cover separately from the secretion line and opens directly to the wound area.

This auxiliary line can also take on the function of a rinse line with which a rinse fluid is delivered to the wound and through the secretion line in order to clean it, for example, from residues of the instillation fluid or the exudate.

WO 2018/130466 A1 discloses, for example, a device for healing wound tissue by way of negative pressure treatment in which instillation fluid is supplied to a wound via an instillation line that opens directly into the wound bed and in which fluids are extracted from the wound bed via a separate secretion line. In addition to the instillation and secretion lines, an auxiliary line can be provided which either opens into the secretion line near the wound bed or directly into the wound bed. The auxiliary line makes it possible to measure the pressure in the secretion line or at the wound or to rinse the secretion line.

EP 3 235 526 A1 discloses a device for negative pressure treatment and instillation of wounds in which a negative pressure unit is connected to a wound dressing via a multi-lumen connection. The pressure in the wound dressing is measured via a lumen of the connection. A primary lumen is used to transport the fluid that is extracted. An instillation container is also connected to the wound dressing via several fluid lumens, where one lumen is used for fluid transport and another for pressure measurement.

A device with a secretion line for extracting wound secretion, an instillation line for supplying instillation fluid, and an auxiliary line for rinsing the secretion line and for measuring pressure is disclosed in US 2019/0275219 A1.

Further devices in which an additional auxiliary line for pressure measurement is connected to the wound dressing are disclosed in EP 2 714 120 B1, US 2011/0178479 A1 and EP 0 865 304 B1. Further, EP 0 880 953 A2 discloses an apparatus for wound treatment comprising an instillation line and a drain line, wherein the instillation line and the drain line are connected to the wound dressing through a single main line. DE 20 2017 003 262 U1 discloses an adapter for connecting different vacuum pumps to a wound dressing.

### DESCRIPTION OF THE INVENTION

It is an object of the present invention to provide an apparatus for negative pressure treatment and instillation of wounds with which a simpler connection of the wound dressing to the apparatus and thereby an easier change of the wound dressing is possible. In addition, cleaning the apparatus is to be made easier also during operation, thereby complying with higher hygienic requirements. Furthermore, disruptions in operation should be detectable, in particular blockage of the fluid connection between the dressing and the apparatus.

To achieve this object, the invention provides an apparatus for negative pressure treatment and instillation of wounds according to claim 1.

The invention is characterized in that two of the following lines, drain line, instillation line, and rinse line, can be combined to form a single-lumen main line and can be connected to the dressing by way of the main line. The two lines are preferably connected to the main line via the connection element. For example, the drain and the instillation line or the instillation and the rinse line are combined by the connection element to form a single-lumen main line and connected to the dressing via the main line. The respective third line is connected separately to the dressing so that the dressing can be connected to the apparatus via no more than two lumens.

As the apparatus comprises each of a drain, instillation and rinse line, two of these lines are combined via the connection element to form the single-lumen main line, while the third line is connected directly to the dressing. In this way, the dressing is connected to the apparatus only via two directly connected lumens.

In conventional apparatuses for negative pressure treatment and instillation of wounds, however, completely separate drain, instillation and rinse lines are provided, which are connected separately to the wound dressing. This means that a total of three fluid lines must be connected to the wound dressing.

In contrast, the apparatus according to the invention only requires the connection of no more than two fluid lines to the wound dressing. In this way, the same type of wound dressing can be used for the combined negative pressure and instillation treatment as for the mere negative pressure or instillation therapy in which only two fluid lines are connected to the wound dressing. The apparatus according to the invention also makes it easier to change the wound dressing because only the connection to two fluid lines needs to be separated. The fact that the main line can be cleaned with the rinse fluid and freed from residues of instillation fluid or the exudate furthermore results in hygienic advantages. Furthermore, blockage in the fluid-carrying system can be determined by measuring the pressure in the rinse line during operation.

Further advantages of the apparatuses according to the invention and preferred embodiments shall be described below.

A "line" within the scope of the present invention, for example a drain or main line, means a fluid connection having a single lumen. A connection with several lumens, for example a double-lumen tube connection, comprises several lines within the scope of this invention.

In the context of the present invention, a connection between two lines or between a line and a connection element, respectively, always means a fluidic connection, i.e. a connection that allows fluid to be forwarded between the connected components.

An instillation fluid can be fed to the wound via the instillation line. The instillation fluid is used, for example, to loosen necrotic tissue from the wound, to clean the wound, and to dilute wound fluid. The instillation fluid therefore contributes to the debridement of the wound. However, the instillation fluid can also be used to introduce a medicinal product or to locally anesthetize the wound area. The instillation fluid can additionally be preconditioned, for example, with a defined temperature, UV radiation and/or a specific chemical composition or mixture. The instillation fluid can be gaseous or liquid. The instillation fluid is preferably an aqueous solution, for example, a physiological saline solution. The instillation fluid can also contain one or more active substances, in particular medicinal active substances for promoting wound healing. The instillation fluid can also be air, air enriched with oxygen, or nitrogen. The instillation fluid can also be an aerosol. The aerosol preferably comprises a carrier gas, such as air or nitrogen, and an aqueous solution atomized therein, such as e.g. an active substance solution.

The instillation line preferably comprises a valve (check valve) with which a backflow of fluid, in particular instillation and rinse fluid from the main line, into the instillation line can be prevented. In an alternative embodiment, the function of this valve can also be assumed by a peristaltic pump arranged in the instillation line, which, for example, squeezes off the fluid line and thereby prevents fluid from flowing back into the instillation line. A peristaltic pump and a check valve can also be provided simultaneously. In certain cases, the sealing effect of the peristaltic pump may not be sufficient so that instillation fluid may inadvertently penetrate the dressing if there is a negative pressure applied in the dressing. To prevent this, an additional controllable valve can be provided that interrupts the fluid connection between the peristaltic pump and the dressing in both directions. This function can also be assumed by the aforementioned check valve.

The instillation line is preferably connected to an instillation container. The latter is preferably connected to the instillation line in an exchangeable manner. The instillation container is used to receive a supply of instillation fluid. The instillation container can be made, for example, of rigid or flexible plastic material. For example, the instillation container is a plastic bag or a plastic bottle. The instillation container is preferably made of transparent material at least in part, in particular transparent plastic material, so that the filling level can be observed from the outside.

In a further embodiment, the instillation container can be arranged relative to the dressing in such a way that the instillation fluid is led to the wound by gravity. For this purpose, the apparatus can comprise a holder for the instillation container, for example, a suspension, with which the instillation container can be fastened at a higher level than the dressing. In this case, the instillation line can comprise a drip counter with which the amount of instillation fluid supplied can be determined. The fluid flow through the instillation line in this embodiment can be controlled by an additionally provided pump described below.

The apparatus comprises a rinse line in addition to the drain and instillation lines. In this embodiment, either the drain and the instillation line or the instillation and the rinse line are preferably connectable to the connection element. These lines are particularly preferably connected to the connection element. Most preferred is the embodiment in which the instillation and the rinse line are connected to the connection element and therefore to the main line, and the drain line is connected to the dressing separately.

A rinse fluid can be supplied to the wound via the rinse line. The rinse fluid can be gaseous or liquid. The rinse fluid is preferably air. The rinse line enables pressure equalization and rinsing of the main line, the dressing, and the drain line. The rinse line is preferably in fluid communication with the ambient air via a controllable ventilation valve.

The rinse line preferably comprises a valve with which a backflow of fluid, in particular instillation and rinse fluid from the main line, into the rinse line can be prevented. This valve can also be a membrane that closes on contact with liquid and interrupts the flow of fluid. Such a membrane can comprise, for example, a material that swells upon contact with moisture and then forms a barrier impermeable to fluid. Alternatively, such a membrane can be configured as a hydrophobic PTFE membrane that only allows air to pass through. Such a configuration of the membrane can also act as a filter to avoid microbiological contamination of the rinse line.

In a preferred embodiment, a pressure sensor is arranged in the rinse line. The negative pressure applied in the rinse line and therefore also the negative pressure applied in the wound dressing can be continuously monitored with the aid of this sensor.

The rinse line also preferably comprises at least one filter unit for removing microbiological contaminants from the rinse fluid. In the event that the rinse line is in fluid communication with the ambient air via a controllable ventilation valve, the ambient air is preferably passed through a filter unit for removing microbiological contaminants from the ambient air. This filter unit is preferably arranged between the ventilation valve and the end of the rinse line which is in contact with the environment. In the event that the rinse line comprises a pressure sensor, an additional filter unit is preferably arranged between the pressure sensor and the end of the rinse line facing the main line.

In a preferred embodiment, the apparatus comprises a pump for generating a fluid flow in the main line. This is preferably a peristaltic pump. A fluid flow is generated in the main line with the aid of the pump for directing the instillation and/or rinse fluid in the direction of the wound. This enables better control when supplying rinse and/or instillation fluid during the treatment. In addition, the flow rate can be adjusted independently of the relative arrangement of the instillation container and the dressing. The pump is therefore preferably also provided if the instillation container is arranged above the dressing in order to generate hydrostatic pressure in the instillation fluid. The pump can be arranged in the main line, the rinse line, the instillation line or the connection element which connects the supply, instillation, and rinse lines to one another. In a preferred embodiment, the pump is arranged in the instillation line and therefore serves to generate a fluid flow in the instillation fluid. A pump can also be provided in the event that the instillation fluid is led to the wound by gravity in order to additionally control the fluid flow.

The pump is used in particular when the instillation and/or rinse fluid is a liquid. In the case of a gaseous fluid, a pump can be dispensed with since a fluid flow can be generated in the gaseous fluid by the negative pressure present in the dressing. In a preferred embodiment, the pump is there used to generate a fluid flow in the preferably liquid instillation fluid, while the fluid flow in the preferably gaseous rinse fluid is generated by negative pressure.

Suitable peristaltic pumps are described, for example, in EP 3 603 689 A1, US 2019/0275218 A1 and WO 2018/172217 A1.

The pump is preferably connected to a control unit with which the pump output can be adjusted in a temporal manner. In addition, the pump can be connected to a measuring device that determines on the basis of the pump output the quantity of instillation and/or rinse fluid supplied.

In a preferred embodiment, the apparatus comprises a unit for controlling the fluid flow through the instillation line with which the flow rate can be controlled in a temporal manner. For example, the apparatus can comprise a controllable valve in the instillation line with which the fluid connection to the installation container can be interrupted. It is also possible to couple the above-mentioned pump for generating a fluid flow in the main line to a control unit for adjusting the pump output.

In a preferred embodiment, the apparatus comprises a unit for controlling the fluid flow through the main line, so that the fluid connection to one of the connected lines can alternately be interrupted. For example, either the instillation fluid or the rinse fluid can be passed through the main line. The unit for controlling the fluid flow can be, for example, two simultaneously controllable valves which respectively open or block the fluid flow through the instillation line or the rinse line, respectively The connection element can also be equipped with a device which alternately opens the fluid connection from the main line to the instillation line or to the rinse line, for example a rotary valve.

A corresponding device can be provided if the connection element is connected to the drain and instillation line. The drain line is used to extract fluids from the wound or from the wound space, respectively. The extracted fluid can be gaseous or liquid. The fluid extracted is in particular body fluid referred to as exudate which is secreted from the wound during the course of the negative pressure treatment. However, the drain line also serves to extract the instillation fluid and the rinse fluid. The fluid flow required for the extraction is generated by the negative pressure applied in the drain line.

A drain valve is preferably arranged in the drain line. The drain valve can be controllable for opening or interrupting the fluid communication within the drain line. This valve makes it possible, for example, to ventilate a collection container possibly connected to the drain line, while a negative pressure is still applied in the dressing. The drain valve is preferably a one-way valve that allows fluid communication in the direction away from the dressing and blocks fluid communication in the direction toward the dressing.

In a preferred embodiment, the apparatus comprises both a drain and an instillation line as well as a rinse line, where two of these lines are connected to the main line via the connection element. The main line and the respective line not connected to the connection element together preferably form a double-lumen fluid line, at least in sections. For example, the instillation line and the rinse line are connected to the main line and the drain line and the main line together form a double-lumen fluid line, at least in sections. This facilitates handling of the apparatus, in particular the connection of the dressing to the main line and drain line, since the double-lumen line can effectively be handled like a single tube connection. For example, the double-lumen line can be connected to the dressing in a single work step by use of a suitable connection element. In addition, a coupling can be provided in the double-lumen line by way of which the latter can be separated from the apparatus.

In a preferred embodiment, the drain line is connected to a collection container for receiving the fluid extracted from the wound. The collection container is preferably made of plastic material, particularly preferably rigid plastic material. The collection container is preferably made of transparent material, at least in part, so that the filling level can be observed from the outside. The collection container preferably comprises a port for connection to the drain line and a further port for connection to the negative pressure unit. The port for connection to the drain line is preferably secured with a drain valve with which a backflow of the extracted fluid from the collection container into the drain line can be prevented. The drain valve can be controllable for opening or interrupting the fluid connection between the dressing and the collection container. The drain valve is preferably a one-way valve that allows fluid communication in the direction toward the collection container and blocks fluid communication in the direction toward the dressing. The port for connection to the negative pressure unit preferably comprises a filter unit for preventing mutual contamination of the collection container and the connection to the negative pressure unit.

The apparatus preferably comprises a pressure sensor for measuring the negative pressure applied in the drain line and/or the collection container. This pressure sensor can be arranged in the drain line, the collection container, or the connection between the collection container and the negative pressure unit. The pressure sensor is particularly preferably arranged in the connection between the collection container and the negative pressure unit.

In one embodiment, the apparatus comprises a bypass line which establishes fluid communication between the drain line and the rinse line. A bypass valve blocking or opening the fluid connection between the drain line and the rinse line is preferably arranged in the bypass line. The bypass line can open into the drain line, for example, at a location between the dressing and the collection container described above. Alternatively, the bypass line can open into the connecting line between the collection container and the negative pressure unit. In the event that the drain line comprises a drain valve as described above, the bypass line preferably opens into the drain line on the side of the drain valve facing away from the dressing. With regard to the rinse line and in the event that it comprises a ventilation valve as described above, the bypass line opens into the rinse line on the side of the ventilation valve facing away from the dressing.

The bypass line enables the drain line and the collection container optionally connected thereto to be ventilated at the beginning of the negative pressure therapy. If the drain line is secured with a drain valve and the rinse line is secured with a ventilation valve, ventilation can take place while the dressing already has negative pressure applied.

The dressing is a dressing suitable for negative pressure treatment of wounds and can seal a wound in a fluid-tight manner, in particular in a gas-tight manner, while forming a wound space, and enables fluid communication between the wound space and a negative pressure source and at least one fluid source. The dressing preferably comprises a self-adhesive cover film with which a wound can be covered in a fluid-tight manner while forming the wound space. The dressing can also comprise a wound cover arranged within the wound space, for example, a wound cover made from a textile or foam material. A wound cover made from open-cell polymer foam material is particularly preferred. The wound cover can be configured having one or more layers. The wound cover preferably comprises an antimicrobial wound contact layer, for example, a layer of polyamide fabric containing silver.

The connection of the supply and drain line to the dressing is established, for example, by pushing the respective ends of the lines under the cover film of the dressing toward the wound space. Alternatively, the dressing can comprise one or more suitable ports which are coupled to the respective ends of the lines. For example, the lines can be plugged onto suitable port elements. In a preferred embodiment, the dressing has a port with two channels via which the supply and the drain line are connected. In an alternative embodiment, the dressing has two separate ports, each with a channel for connection to the supply line and the drain line, respectively.

The negative pressure unit for generating the negative pressure is preferably a vacuum pump. A negative pressure is generated in the drain line with the negative pressure unit and continues into the wound space formed by the fluid-tight dressing and the main line connected thereto. The negative pressure then prevailing in the wound space promotes wound healing and also allows fluid to be extracted from the wound space. The negative pressure unit is preferably a diaphragm pump. A diaphragm pump is particularly suitable because it creates an oscillating pressure curve that has a positive effect on wound healing. As an alternative thereto, precautions can also be provided to prevent the negative pressure from oscillating so that the negative pressure prevailing in the wound space is constant over time. The apparatus preferably comprises a control unit with which the suction power of the negative pressure unit can be time-controlled. This enables, for example, intermittent extraction of fluid from the wound space. It has been shown that intermittent extraction can have a positive effect on wound healing.

The aforementioned control units, in particular the control units for adjusting the pump output and the suction power as well as the unit for controlling the fluid flow through the main line, are preferably programmable so that the operation of the apparatus is controlled according to a predetermined temporal sequence. The control units mentioned can additionally monitor one or more operating parameters of the apparatus and control the operation of the apparatus, in particular the pump output, the suction power, and the fluid flow through the main line, in dependence of these operating parameters. For example, the negative pressure in the dressing and/or one of the connected lines, or the filling level of one of the fluid containers connected to the apparatus are monitored. For this purpose, the control units mentioned can also be connected to a central programmable control unit or combined to form such a central unit, respectively.

In a preferred embodiment, the apparatus comprises a housing which accommodates the negative pressure unit. The housing can also accommodate a power supply, in particular a power supply unit with a connection to an external power source or a rechargeable battery. Alternatively, such a power supply unit can be arranged outside the housing. In a preferred embodiment, the apparatus comprises a rechargeable battery, in particular a lithium-ion accumulator, which is arranged within the housing and can be charged via a power connection accessible on the outside of the housing. This has the advantage that the apparatus is portable and can be used independent of a permanently installed electricity network.

In a preferred embodiment, at least a part of the rinse line runs within the housing, where an optional pressure sensor connected to the rinse line can also be arranged within the housing. In addition, the housing preferably comprises a ventilation device arranged on the outside with which the rinse line is brought into fluid communication with the ambient air.

In a preferred embodiment, the instillation line runs outside the housing. If the instillation line is connected to the connection element, a port for connecting the instillation line to the connection element can be provided on the outside of the housing. The instillation line preferably connects the instillation container to the housing.

The instillation container can also be detachably connected to the housing so that it forms a unit with the housing when connected. For example, the instillation container can form a unit together with the collection container for receiving the fluid extracted from the wound, where this unit is detachably connected to the housing. In a further embodiment, the instillation container is arranged within the housing.

In a preferred embodiment, the connection element for connecting the instillation and rinse line is arranged within the housing. In this case, the housing can comprise a port on the outside for connecting the connection element to the main line.

An optionally available collection container for receiving the fluid extracted from the wound is preferably detachably connected to the housing. In the connected state, the collection container preferably forms a unit with the housing. The collection container is preferably attached to the housing by way of a snap connection.

The invention further relates to a method for operating the apparatus described above for negative pressure treatment and instillation of wounds. The method comprises the following steps:
a) filling the dressing with instillation fluid from the main line,
b) passing a rinse fluid through the rinse line, the main line, the dressing and the drain line during simultaneous, continuous, or intermittent operation of the negative pressure unit.

Step b) is made possible by the apparatus according to the invention in which the rinse line and instillation line jointly open into the main line. This makes it possible not only to rinse the dressing and the drain line in step b) but simultaneously also the instillation line.

The rinse fluid is passed through in step b) by way of negative pressure. For this purpose, a negative pressure is generated in the dressing by the negative pressure unit. For this purpose, the negative pressure unit is operated at the same time as the rinse fluid is passed through. The negative pressure unit is operated either continuously, i.e. with substantially constant output, or intermittently, i.e. with intermittent or decreasing output.

In one embodiment of the method, step a) is preceded by the following steps:
a1) building up negative pressure in the dressing by operating the negative pressure unit,
a2) filling the instillation line and the main line with instillation fluid.

The fluid connection in the drain line is preferably interrupted between steps a1) and a2). A collection container which may be connected to the drain line can be ventilated in this manner while negative pressure continues to be applied in the dressing or while the dressing is being filled with instillation fluid, respectively.

In addition, the negative pressure can be measured following step a1) for a predetermined period of time in order to check the tightness of the dressing.

The negative pressure generated in the dressing in step a1) is used in subsequent steps a2) and a) to generate a fluid flow in the instillation fluid with which the instillation fluid is first sucked into the main line and then into the dressing.

In a further embodiment, step b) is followed by the following additional steps:
c) building up negative pressure in the dressing by operating the negative pressure unit,
d) passing a rinse fluid through the rinse line, the main line, the dressing and the drain line.

Step c) represents the negative pressure treatment. In this step, any instillation fluid present in the dressing is extracted. The negative pressure unit is preferably operated intermittently in step c) in order to generate intervals of higher or lower negative pressure in the dressing. It is also possible to carry out steps c) and d) several times in succession. In a further embodiment, steps a) to d) are repeated several times. In this embodiment, the quantity of instillation fluid in the dressing can additionally be determined when step a) is carried out for the first time. The determination is preferably made visually by the user of the apparatus. The fill quantity thus determined indicates the maximum filling volume of the dressing. With subsequent repetitions of step a), the dressing is respectively filled with the previously determined fill quantity of instillation fluid, without the fill quantity having to be determined anew.

If, in this embodiment, the negative pressure is measured for a predetermined period of time prior to step a) in order to verify the tightness of the dressing, it is sufficient to carry out this measurement before step a) is carried out for the first time.

### DESCRIPTION OF THE DRAWINGS

Preferred embodiments of the invention shall be described below with reference to the drawings which are only used for explanation purposes and are not to be interpreted as being restrictive, where, in the drawings:
- Fig. 1: shows a schematic view of an apparatus according to the invention;
- Figs. 2A-C: show schematic views of alternative embodiments of the apparatus according to the invention;
- Fig. 3: shows a schematic view of an alternative embodiment of the apparatus according to the invention and
- Figs. 4A-B: show schematic views of alternative embodiments of the apparatus according to the invention.
- Fig. 5: shows a schematic view of a variant of the apparatus according to the invention;
- Fig. 6: shows a schematic view of a variant of the apparatus according to the invention.

### DESCRIPTION OF PREFERRED EMBODIMENTS

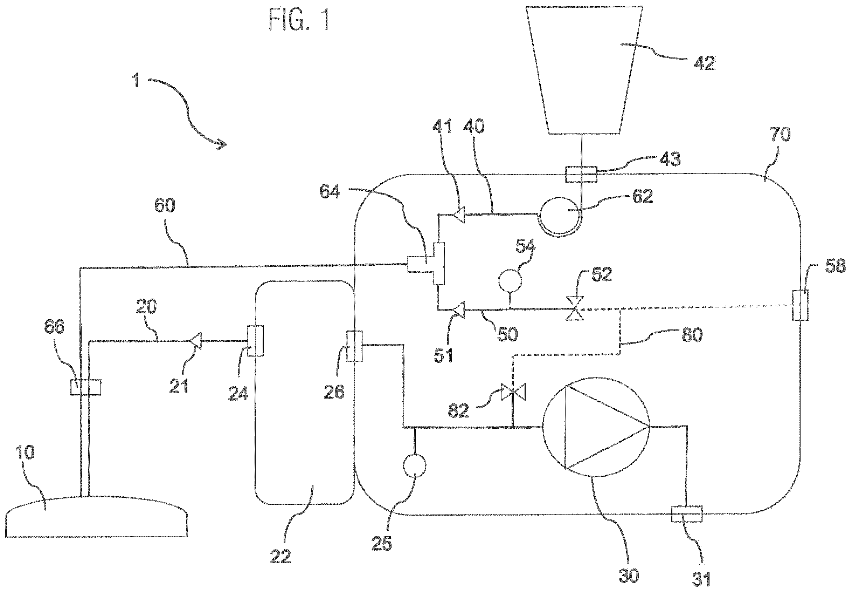

The apparatus 1 shown in Figure 1 comprises a dressing 10 for covering a wound. The dressing 10 typically comprises a flexible elastic plastic cover with which a wound can be covered in a fluid-tight manner, i.e. i.e. in a gas and liquid-tight manner, thus forming a wound space. The dressing 10 further comprises a wound cover, not shown in the drawing, which is arranged within the wound space.

The dressing 10 is connected via a port - not shown in the drawing - in a fluid-conducting manner to a drain line 20 and a main line 60. The port facilitates the connection and removal of the drain line 20 and the main line 60 to and from the dressing 10.

The drain line 20 opens with a second end of the line facing away from the dressing into the interior of a collection container 22. The collection container 22 is used to receive the fluid extracted from the wound. The fluid is extracted by negative pressure which is generated by a negative pressure unit 30 that is connected to the collection container in a fluid-conducting manner. The negative pressure unit is arranged within a housing 70.

In the embodiment shown in Figure 1, the collection container 22 is attached to the housing 70. It can be designed as a disposable item and removed from the housing and disposed of together with the collected fluid once the maximum filling level has been reached. The collection container, however, can also be reusable in that it can be removed, cleaned and reattached to the housing. The collection container 22 is preferably attached to the housing 70 by way of a snap connection.

The drain line 20 is connected to the collection container 22 by way of a detachable port 24. Provided on the side of the port facing away from the collection container 22 is a drain valve 21 which blocks the fluid flow in the direction of the dressing, but allows fluid flow in the direction of the collection container.

The apparatus also comprises a housing 70 which accommodates the negative pressure unit 30. The negative pressure unit 30 is in fluid communication with the collection container 22 and can therefore generate a negative pressure in the collection container 22 which continues via the drain line 20 to the wound space. Arranged between the collection container 22 and the negative pressure unit 30 is a filter 26 which prevents microbial contamination of the collection container 22 or the negative pressure unit 30. A pressure sensor 25 is additionally arranged between filter 26 and negative pressure unit 30.

The negative pressure unit 30 is also in fluid communication with an outlet opening 31 on the outer side of the housing 70.

The main line 60 is connected to a connection element 64 which is preferably arranged within the housing. The connection element 64 is, for example, a T- or Y-shaped connection member with a total of three ports for the main line 60, the instillation line 40, and the rinse line 50. The connection element 64 can be arranged such that the port for the main line 60 is exposed on the outer side of the housing 70 and accessible from the outside. In this case, the main line can be detachably connected to the connection element 64 so that the main line 60 can easily be exchanged.

The drain line 20 and the main line 60 both run through a coupling 66. Between the coupling 66 and the dressing 10, the drain line 20 and the main line 60 together form a double-lumen tube connection. The coupling 66 allows for simple connection and removal of the double-lumen tube connection to or from the apparatus 1. The double-lumen tube connection forms an entity with the dressing 10. This entity can be designed as a disposable item that can be disposed of after use.

The connection element 64 is connected with the second of its ports to the instillation line 40. A valve 41 can be arranged in the instillation line 40 in order to prevent a backflow of instillation or rinse fluid into the instillation line 40. The valve can additionally have the function of preventing the instillation fluid from inadvertently passing through in the direction of the dressing 10. The instillation line 40 is connected to an instillation container 42 which is arranged outside the housing 70 in the apparatus shown in the drawing. A port 43 with which the instillation line 40 and the instillation container 42 can be connected to one another is arranged on the outer side of the housing 70. The port 43 is preferably detachable so that the instillation container 42 can be exchanged easily.

In the apparatus shown in the drawing, the instillation container 42 is a plastic bag or a plastic bottle arranged upside down and is attached above the housing 70 and above the dressing 10. In this manner, a fluid flow is generated by hydrostatic pressure.

It is also possible that the instillation container 42, similar to the collection container 22, is attached to the housing 70 such that it can be coupled thereonto. In this case, the tube connection between the port 43 and the instillation container 42 is omitted.

A peristaltic pump 62 which generates a fluid flow in the instillation fluid is arranged at the instillation line 40. The peristaltic pump 62 is controllable so that the fluid flow can be varied in the course of the treatment. Peristaltic pump 62 is arranged on the side of valve 41 facing away from connection element 64.

In the embodiment shown in the drawing, the peristaltic pump 62 is arranged within the housing 70. However, it is also possible to mount the peristaltic pump 62 on the outer side of the housing 70 or in or on the collection container 22, or to design it as a separate module.

The connection element 64 is connected with the third of its ports to the rinse line 50. A valve 51 can be arranged in the rinse line 50 in order to prevent a backflow of instillation or rinse fluid into the rinse line 50. The valve 51 is preferably a membrane which seals on contact with moisture. Arranged on the side of the valve 51 facing away from the connection element 64 is a pressure sensor with which the negative pressure in the rinse line 50, the main line 60 connected thereto, and the wound space can be measured. Arranged on the side of the pressure sensor 54 facing away from the connection element 64 is a ventilation valve 52 that can open or interrupt the fluid communication through the rinse line 50. The rinse line 50 is in fluid communication with the ambient air via a port 58. The rinse line 50 and the main line 60 connected thereto can be ventilated in this way. The port 58 comprises a filter element for preventing contamination of the rinse line 50 with microorganisms from the ambient air.

The rinse line 50 is in fluid communication via a bypass line 80 with the negative pressure unit 30 and the fluid collection container 22 connected thereto. The bypass line 80 can be opened or closed by way of a bypass valve 82. Direct ventilation of the negative pressure unit 30 and the fluid collection container 22 is also possible in this manner. The valve 21 prevents simultaneous ventilation of the drain line 20 and the wound space. The negative pressure unit 30 and the collection container 22 can be ventilated while negative pressure is simultaneously applied in the wound space.

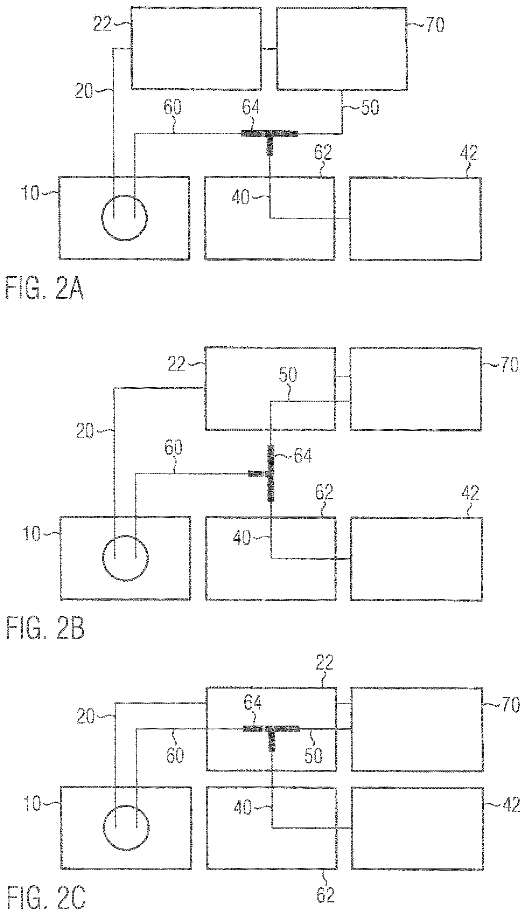

Figures 2A to 2C show different variants of the apparatus according to the invention in a schematic view. Like the embodiment described above, the embodiments according to these variants comprise a housing 70 which accommodates a negative pressure unit (not shown in the drawings). The negative pressure unit is in fluid communication with a collection container 22. The collection container 22 is connected to the dressing 10 via the drain line 20. The dressing 10 is connected to an instillation line 40 and a rinse line 50 via the main line 60 and a connection element 64. The rinse line 50 respectively opens into the housing 70.

In these variants, the dressing 10 has a single port to which both the drain line 20 and the main line 60 are connected.

Unlike the embodiment previously shown, the peristaltic pump 62 in this variant is arranged outside the housing 70 and forms a separate module of the apparatus. This module can be configured, for example, like in US 2019/0275219 A1.

The variants each differ in the arrangement of the connection element 64. According to Figures 2A and 2B, the connection element 64 is arranged between the housing 70, the collection container 22, and the peristaltic pump 62. According to Figure 2A, both the instillation line 40 and the rinse line 50 run outside the collection container 22. According to Figure 2B, the rinse line 50 runs through the collection container 22. In the variant according to Figure 2C, the connection element 64 is arranged within the collection container 22 so that the rinse line 50 and the instillation line run within the collection container 22, at least in sections.

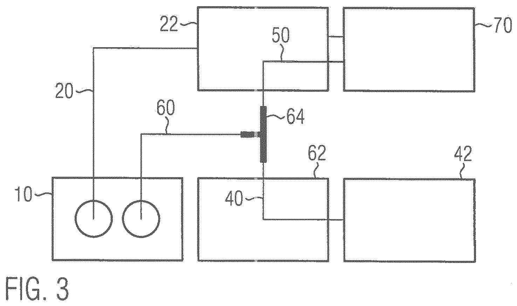

Figure 3 shows a further variant of the apparatus according to the invention in a schematic view. This variant corresponds to the embodiment according to Figure 2B, but here the dressing 10 has two separate ports for the drain line 20 and the main line 60, respectively.

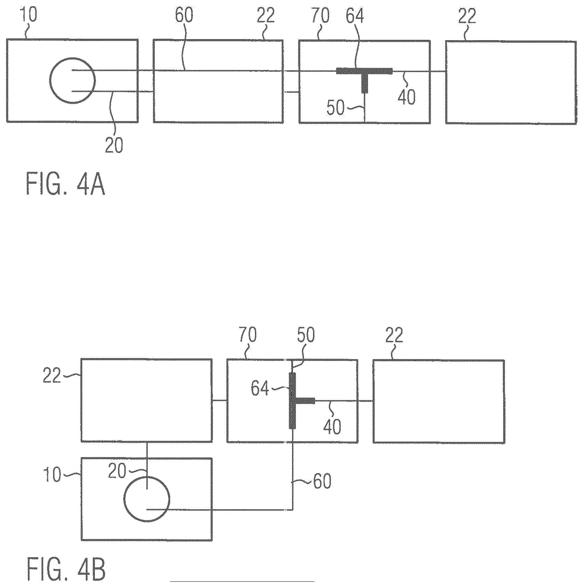

Figures 4A and 4B show further variants of the apparatus according to the invention in a schematic view. In these variants, the housing 70 accommodates both the peristaltic pump (not shown in the drawings) as well as the negative pressure unit. In contrast to the variants shown above, the connection element 64 is additionally arranged within the housing 70. The two variants in this respect correspond to the embodiment according to Figure 1. The difference between the two variants arises from the fact that the main line according to Figure 4A runs through the collection container 22, whereas according to Figure 4B it runs outside the collection container 22.

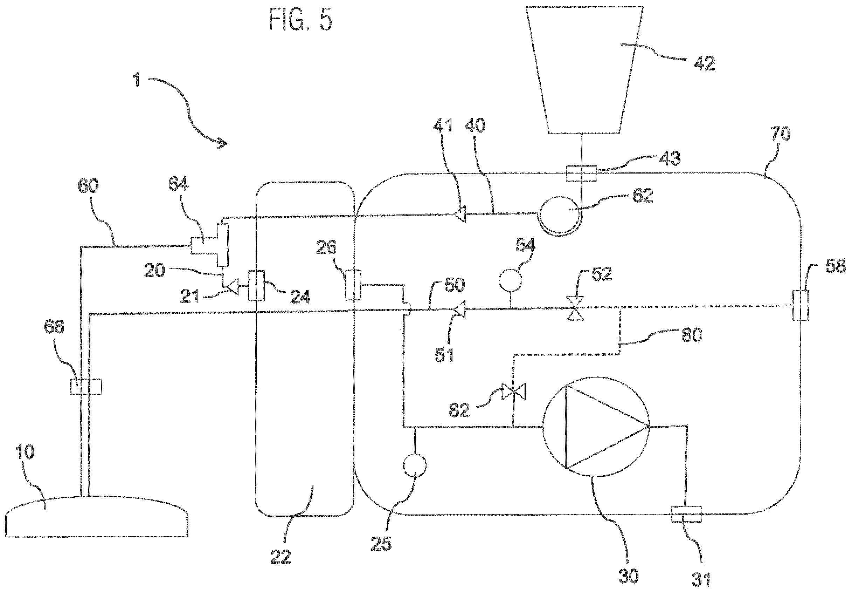

Figure 5 shows a further variant of the apparatus according to the invention. This variant corresponds to the embodiment shown in Figure 1 apart from the following differences. Unlike in the embodiment shown in Figure 1, the connection element 64 in Figure 5 is connected to the instillation line 40 and the drain line 20. As a result, the instillation line 40 and the drain line 20 are jointly connected to the single-lumen main line 60. In this way, both the instillation fluid and the wound secretion are extracted from the wound through the lumen of the main line 60 The connection element preferably has a device with which a fluid connection to the drain line 20 or to the instillation line 40 can alternately be established and the fluid connection to the respective other line can be interrupted.

In this embodiment, the rinse line 50 forms a separate line which is led to the dressing 10 separately from the drain line 20 and the instillation line 40.

In this embodiment, the pressure is measured by the pressure sensor 54 arranged in the rinse line 50 and/or the pressure sensor 25 arranged in the fluid connection between the collection container 22 and the negative pressure unit 30.

In one section, the rinse line 50 and the main line 60 form a double-lumen tube connection. This section extends between the coupling 66 and the dressing 10.

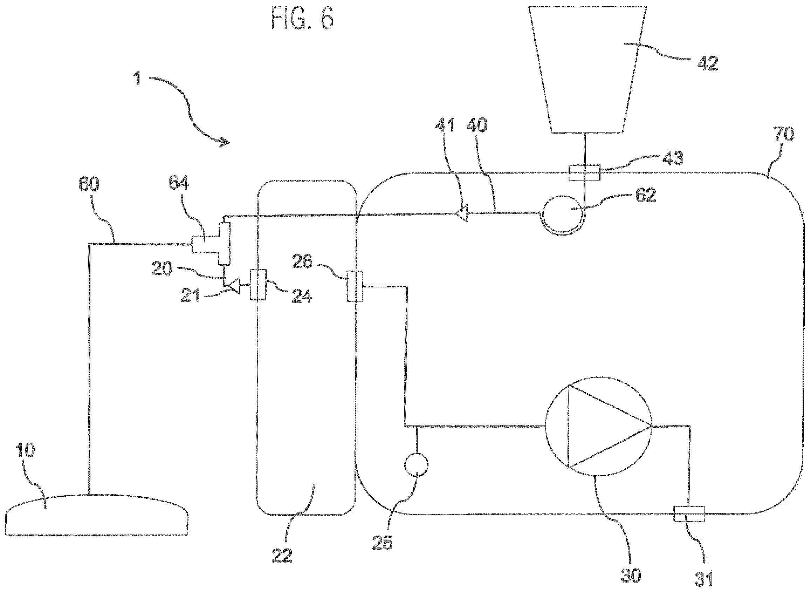

Figure 6 shows a further variant of the apparatus according to the invention. This variant is similar to the variant according to Figure 5, except for the fact that the apparatus according to Figure 6 comprises no rinse line. Like in Figure 5, however, the apparatus comprises a drain line 20 and an instillation line 40, both of which are connected to the connection element 64 and via the latter to the main line 60. In this way, both the instillation fluid and the wound secretion are extracted from the wound through the lumen of the main line 60 In this embodiment, the dressing 10 is connected to only a single line, the main line 60.

In this embodiment, the pressure is measured by the pressure sensor 25 arranged in the fluid connection between the collection container 22 and the negative pressure unit 30.

### List of reference numerals

- 1: apparatus
- 10: dressing
- 20: drain line
- 21: drain valve
- 22: collection container
- 24: port
- 25: pressure sensor
- 26: filter
- 30: negative pressure unit
- 31: outlet opening
- 40: instillation line
- 41: valve
- 42: instillation container
- 43: port
- 50: rinse line
- 51: valve
- 52: ventilation valve
- 54: pressure sensor
- 56: ventilation device
- 58: filter
- 60: main line
- 62: pump
- 64: connection element
- 66: coupling
- 70: housing
- 80: bypass line
- 82: bypass valve

## Claims

1. Apparatus (1) for negative pressure treatment and instillation of wounds, comprising
a dressing (10) for covering a wound in a fluid-tight manner,
a drain line (20) for extracting fluid from said wound,
a negative pressure unit (30) for generating negative pressure in said drain line,
an instillation line (40) for providing an instillation fluid,
an rinse line (50) for providing a rinse fluid,
a main line (60) connected to said dressing (10),
**characterized in that**,
said main line (60) comprises a single lumen and can be connected via a connection element (64) to two of the following lines, drain line (20), instillation line (40), and rinse line (50), while the third line is connected directly to the dressing (10).

2. Apparatus according to claim 1, where
said main line (60) can be connected via said connection element (64) either to said drain line (20) and said instillation line (40) or to said instillation line (40) and said rinse line (50).

3. Apparatus according to claim 2, where
said apparatus (1) comprises said rinse line (50) and
said main line (60) can be connected via said connection element (64) to said instillation line (40) and said rinse line (50).

4. Apparatus according to one of the preceding claims, further comprising
a pressure sensor (25, 54) for measuring the pressure in said drain line (20), said instillation line (40), said rinse line (50) and/or said main line (60).

5. Apparatus according to one of the preceding claims, further comprising
a valve (41) that is arranged in said instillation line (40) and prevents a backflow of fluid into said instillation line (40).

6. Apparatus according to one of the preceding claims, further comprising
an instillation container (42) connected to said instillation line (40) for receiving an instillation fluid.

7. Apparatus according to one of the preceding claims, further comprising
a valve (51) that is arranged in said rinse line (50) for preventing a backflow of fluid into said rinse line (50).

8. Apparatus according to one of the preceding claims, further comprising
a ventilation valve (52) arranged in said rinse line (50) with which the fluid communication within said rinse line (50) can be interrupted.

9. Apparatus according to one of the preceding claims, further comprising
a pump (62) arranged in said instillation line (40), said rinse line (50), or said main line (60) for generating a fluid flow.

10. Apparatus according to one of the preceding claims, further comprising
a unit for controlling the fluid flow through said main line (60) so that either the instillation fluid or the rinse fluid is alternately passed through said main line (60).

11. Apparatus according to one of the preceding claims, further comprising
a collection container (22) connected to said drain line (20) for receiving the fluid extracted from said wound.

12. Apparatus according to one of the preceding claims, further comprising
a bypass line (80) which establishes fluid communication between said rinse line (50) and said negative pressure unit (30).

## Patentansprüche

1. Vorrichtung (1) zur Unterdruckbehandlung und Instillation von Wunden, umfassend:
einen Verband (10) zur fluiddichten Abdeckung einer Wunde,
eine Abflussleitung (20) zum Absaugen von Fluid von der Wunde,
ein Unterdruckaggregat (30) zum Erzeugen von Unterdruck in der Abflussleitung,
eine Instillationsleitung (40) zum Bereitstellen eines Instillationsfluids,
eine Spülleitung (50) zum Bereitstellen eines Spülfluids,
eine mit dem Verband (10) verbundene Hauptleitung (60),
**dadurch gekennzeichnet, dass**
die Hauptleitung (60) ein einzelnes Lumen umfasst und über ein Anschlusselement (64) mit zwei der folgenden Leitungen, Abflussleitung (20), Instillationsleitung (40) und Spülleitung (50), verbindbar ist, während die jeweils dritte Leitung direkt mit dem Verband (10) verbindbar ist.

2. Vorrichtung nach Anspruch 1, wobei
die Hauptleitung (60) über das Anschlusselement (64) entweder mit der Abflussleitung (20) und der Instillationsleitung (40) oder mit der Instillationsleitung (40) und der Spülleitung (50) verbindbar ist.

3. Vorrichtung nach Anspruch 2, wobei
die Vorrichtung (1) die Spülleitung (50) aufweist und
die Hauptleitung (60) über das Anschlusselement (64) mit der Instillationsleitung (40) und der Spülleitung (50) verbindbar ist.

4. Vorrichtung nach einem der vorhergehenden Ansprüche, weiterhin umfassend:
einen Drucksensor (25, 54) zum Messen des Drucks in der Abflussleitung (20), der Instillationsleitung (40), der Spülleitung (50) und/oder der Hauptleitung (60).

5. Vorrichtung nach einem der vorhergehenden Ansprüche, weiterhin umfassend:
ein Ventil (41), das in der Instillationsleitung (40) angeordnet ist und einen Rückfluss von Fluid in die Instillationsleitung (40) verhindert.

6. Vorrichtung nach einem der vorhergehenden Ansprüche, weiterhin umfassend:
einen mit der Instillationsleitung (40) verbundenen Instillationsbehälter (42) zum Aufnehmen eines Instillationsfluids.

7. Vorrichtung nach einem der vorhergehenden Ansprüche, weiterhin umfassend:
ein in Ventil (51), das der Spülleitung (50) angeordnet ist und einen Rückfluss von Fluid in die Spülleitung (50) verhindert.

8. Vorrichtung nach einem der vorhergehenden Ansprüche, weiterhin umfassend:
ein in der Spülleitung (50) angeordnetes Belüftungsventil (52), mit dem die Fluidkommunikation innerhalb der Spülleitung (50) unterbrochen werden kann.

9. Vorrichtung nach einem der vorhergehenden Ansprüche, weiterhin umfassend:
eine in der Instillationsleitung (40), der Spülleitung (50) oder der Hauptleitung (60) angeordnete Pumpe (62) zum Erzeugen eines Fluidstroms.

10. Vorrichtung nach einem der vorhergehenden Ansprüche, weiterhin umfassend:
eine Einheit zum Steuern des Fluidstroms durch die Hauptleitung (60), so dass alternativ entweder das Instillationsfluid oder das Spülfluid durch die Hauptleitung (60) geleitet wird.

11. Vorrichtung nach einem der vorhergehenden Ansprüche, weiterhin umfassend:
einen mit der Abflussleitung (20) verbundenen Sammelbehälter (22) zum Aufnehmen des von der Wunde abgesaugten Fluids.

12. Vorrichtung nach einem der vorhergehenden Ansprüche, weiterhin umfassend:
eine Bypassleitung (80), die Fluidkommunikation zwischen der Spülleitung (50) und dem Unterdruckaggregat (30) herstellt.

## Revendications

1. Appareil (1) pour traitement par pression négative et instillation de plaies, comprenant
un pansement (10) pour recouvrir une plaie d'une manière hermétique aux fluides,
une conduite de drain (20) pour extraire du fluide de ladite plaie,
une unité de pression négative (30) pour générer une pression négative dans ladite conduite de drain,
une conduite d'instillation (40) pour fournir un fluide d'instillation,
une conduite de rinçage (50) pour fournir un fluide de rinçage,
une conduite principale (60) raccordée audit pansement (10),
**caractérisé en ce que**,
ladite conduite principale (60) comprend une lumière unique et peut être raccordée par l'intermédiaire d'un élément de raccordement (64) à deux des conduites suivantes, conduite de drain (20), conduite d'instillation (40), et conduite de rinçage (50), tandis que la troisième conduite est raccordée directement au pansement (10).

2. Appareil selon la revendication 1, où
ladite conduite principale (60) peut être raccordée par l'intermédiaire dudit élément de raccordement (64) soit à ladite conduite de drain (20) et à ladite conduite d'instillation (40) soit à ladite conduite d'instillation (40) et à ladite conduite de rinçage (50).

3. Appareil selon la revendication 2, où
ledit appareil (1) comprend ladite conduite de rinçage (50) et
ladite conduite principale (60) peut être raccordée par l'intermédiaire dudit élément de raccordement (64) à ladite conduite d'instillation (40) et à ladite conduite de rinçage (50).

4. Appareil selon l'une des revendications précédentes, comprenant en outre
un capteur de pression (25, 54) pour mesurer la pression dans ladite conduite de drain (20), ladite conduite d'instillation (40), ladite conduite de rinçage (50) et/ou ladite conduite principale (60).

5. Appareil selon l'une des revendications précédentes, comprenant en outre
une valve (41) qui est agencée dans ladite conduite d'instillation (40) et qui empêche un reflux de fluide dans ladite conduite d'instillation (40).

6. Appareil selon l'une des revendications précédentes, comprenant en outre
un récipient d'instillation (42) raccordé à ladite conduite d'instillation (40) pour recevoir un fluide d'instillation.

7. Appareil selon l'une des revendications précédentes, comprenant en outre
une valve (51) qui est agencée dans ladite conduite de rinçage (50) pour empêcher un reflux de fluide dans ladite conduite de rinçage (50).

8. Appareil selon l'une des revendications précédentes, comprenant en outre
une valve de ventilation (52) agencée dans ladite conduite de rinçage (50) avec laquelle la communication de fluide à l'intérieur de ladite conduite de rinçage (50) peut être interrompue.

9. Appareil selon l'une des revendications précédentes, comprenant en outre
une pompe (62) agencée dans ladite conduite d'instillation (40), ladite conduite de rinçage (50), ou ladite conduite principale (60) pour générer un écoulement de fluide.

10. Appareil selon l'une des revendications précédentes, comprenant en outre
une unité de commande de l'écoulement de fluide à travers ladite conduite principale (60) de sorte que soit le fluide d'instillation soit le fluide de rinçage passe alternativement à travers ladite conduite principale (60).

11. Appareil selon l'une des revendications précédentes, comprenant en outre
un récipient de collecte (22) raccordé à ladite conduite de drain (20) pour recevoir le fluide extrait de ladite plaie.

12. Appareil selon l'une des revendications précédentes, comprenant en outre
une conduite de dérivation (80) qui établit une communication fluidique entre ladite conduite de rinçage (50) et ladite unité de pression négative (30).
